# EUROPEAN PATENT SPECIFICATION

(11) **EP 0 458 537 B1**
(45) Date of publication and mention of the grant of the patent: **31.03.1999**
(21) Application number: 91304449.1
(22) Date of filing: 17.05.1991
(51) Int. Cl.: C07K 5/08, C07K 5/06, A61K 38/55, A61K 38/00

(54) **Use of a therapeutic compound for the treatment of bronchitis**
Verwendung einer therapeutischen Zusammensetzung zur Behandlung von Bronchitis
Usage d'une composition thérapeutique pour le traitement de la bronchite

(30) Priority: 24.05.1990 US 528657
(43) Date of publication of application: 27.11.1991
(73) Proprietor: ZENECA LIMITED, London W1Y 6LN (GB)
(72) Inventor: Glass, Mitchell, Wilmington, DE 19806 (US); Williams, Joseph Campbell, Elkton, MD 21921 (US)
(74) Representative: Smith, Stephen Collyer

(56) References cited:
- EP-A- 0 189 305
- EP-A- 0 276 101
- EP-A- 0 291 234
- EP-A- 0 337 704
- EP-A- 0 345 906
- EP-A- 0 369 391
- EP-A- 0 402 068
- WO-A-86/00077
- AM. REV. RESP. DIS., vol.144, no., 1991, page 875 - 883, ''

## Description

This invention describes a novel use of 4-(4-chlorophenylsulphonylcarbamoyl)-benzoyl-L-valyl-L-proline 1(RS)-(1-trifluoroacetyl-2-methylpropyl)-amide, or a pharmaceutically acceptable salt thereof, in the symptomatic treatment of bronchitis. (Although the aforementioned compound is named here as 1(RS), the invention described herein includes any ratio of the 1(R)- and 1(S)-isomers of the above named compound, or the pharmaceutically acceptable salts thereof.)

Bronchitis is an inherited or acquired, acute or chronic disease characterized by mucus hypersecretion, generally accompanied by poor clearance of the airway secretions, obstruction of airflow and sometimes chronic bacterial infection of the airways.

Accordingly, the present invention provides the use of 4-(4-chlorophenylsulphonylcarbamoyl)benzoyl-L-valyl-L-proline 1(RS)-(1-trifluoroacetyl-2-methylpropyl)amide, or a pharmaceutically acceptable salt thereof, in the manufacture of a novel medicament for use in the treatment of bronchitis.

As another aspect of the invention, there is provided the use of 4-(4-chlorophenylsulphonylcarbamoyl)benzoyl-L-valyl-L-proline 1(RS)-(1-trifluoroacetyl-2-methylpropyl)amide, or a pharmaceutically acceptable salt thereof, in the manufacture of a novel medicament for use in the treatment of bronchitis wherein in addition another pharmacological agent indicated for the treatment of bronchitis is used in the manufacture of the medicament. Such agents include, but are not limited to, antibiotics, bronchodilators, corticosteroids, oxygen, mucolytics, and mucorheologic agents.

Suitable pharmaceutically acceptable salts of 4-(4-chlorophenylsulphonylcarbamoyl)benzoyl-L-valyl-L-proline 1(RS)-(1-trifluoroacetyl-2-methylpropyl)amide (hereafter referred to as "the Compound") include, for example, those described in United States patent 4,910,190, for example, alkali metal and alkaline earth metal salts (such as sodium, potassium, calcium or magnesium salts), ammonium salts, and salts with organic bases affording a pharmaceutically acceptable cation. A preferred salt of the Compound for use for treatment of bronchitis is, for example, a sodium or potassium salt.

The Compound and its production are described in United States patent 4,910,190 where it was referred to as 3(RS)-[4-[(4-chlorophenyl)sulfonylaminocarbonyl]phenylcarbonyl]-L-valyl-N-[3-(1,1,1-trifluoro-4-methyl-2-oxopentyl)]-L-prolinamide, but the name given hereinabove is now preferred. It is noted that Dess-Martin periodinane, described as the preferred oxidant and used in the final step for the production of the Compound in Examples 104 and 121, may in certain circumstances constitute an explosive hazard. Accordingly, it may be preferred to use an alternative oxidant for preparing the ketone from the corresponding alcohol. Alternative methods which may be useful include the use of oxalyl chloride, dimethyl sulfoxide and a tertiary amine (with the best results being obtained with 10-20 equivalents of oxidizing agent); the use of acetic anhydride and dimethyl sulfoxide; the use of chromium trioxide pyridine complex in methylene chloride; and the use of alkaline potassium permanganate solution. For example, the Compound may be obtained from the corresponding alcohol in approximately 60% yield using two equivalents of the latter oxidant.

The medicament obtained by the novel use of the Compound will generally be in the form of a conventional pharmaceutical composition, for example, as generally described in United States patent 4,910,190, and preferably as an aerosol. A formulation providing a solution containing a concentration of 10 mg/mL of the Compound and suitable for use with a nebulizer or as an injectable solution is described below in Example 1. A suitable nebulizer for use is, for example, a RETEC (trademark) nebulizer, in which the solution is nebulized with compressed air.

In general, the medicament obtained by the novel use of the Compound will be such that the Compound will be administered to humans at a daily dose in the range of, for example, 5 to 100 mg of the Compound by aerosol or 50 to 1000 mg intravenously, or a combination of the two. However, it readily will be understood that it may be necessary to vary the dose of the Compound administered in accordance with well known medical practice to take account of the nature and severity of the bronchitis under treatment, concurrent therapy, and the age, weight and sex of the patient receiving treatment. It similarly will be understood that generally equivalent amounts of a pharmaceutically acceptable salt of the Compound also may be used.

The utility of the Compound, or a pharmaceutically acceptable salt thereof, in the symptomatic treatment of bronchitis may be demonstrated using standard clinical study protocols, for example as described below in Study A and Study B, in which improvement in clinical or biochemical parameters may be measured.

Study A in bronchitis is a randomized, double blind, parallel study in 10 to 20 adult patients assigned to receive 35 mg/day of the Compound or vehicle (placebo) to be administered by aerosol inhalation for two to three weeks. A formulation as described in Example 1 may be used for the treatment group, and a similar formulation without the Compound for the vehicle (control) group. The RETEC (trademark) nebulizer is filled with approximately 3.5 mL of the study medication or vehicle (control), as appropriate. The solution in the nebulizer is nebulized with compressed air. The patient breathes normally (tidal volume) for eight minutes with the nebulizer in his mouth. Clinical endpoints include sputum production, spirometry and peak flow, using standard clinical methods in accord with American Thoracic Society standards. Improvements in clinical variables, such as symptoms (using diary cards), sputum production, FEV₁ (forced expiratory volume in one second), and FVC (forced vital capacity), are determined by standard methods of statistical analysis.

Study B in bronchitis is a randomized, double-blind, parallel study in 10 to 20 adult patients assigned to receive the Compound administered at 350 mg/day (for example, 35 mL of the formulation of Example 1) or a corresponding amount of vehicle (placebo) by intravenous infusion for 3 to 4 days, followed by aerosol inhalation at 35 mg/day for 2-3 weeks (as described in Study A). Bronchoalveolar lavage is performed at the start of the study and at the completion of the intravenous and aerosol phases. Clinical variables examined include symptoms, sputum production, spirometry and peak flow, measured and analyzed as described for Study A. Optional biochemical studies include measurements of bronchoalveolar lavage fluid activity on neutrophil phagocytosis and killing of P. aeruginosa, analyzed by standard methods.

The following non-limiting Example illustrates a typical formulation of the Compound for use in the method of treatment provided by the invention.

### Example 1

This example provides a formulation for 4-(4-chlorophenylsulphonylcarbamoyl)benzoyl-L-valyl-L-proline 1(RS)-(1-trifluoroacetyl-2-methylpropyl)amide, listed as a THERAPEUTIC COMPOUND which provides a strength of 10 mg/mL in phosphate-buffered saline and is suitable for a nebulizer solution or for an injectable solution. A corresponding PLACEBO formulation is also provided. The prepared solutions are preferably sealed in ampules of a convenient size, for example 5 mL, and stored with refrigeration until use.

| INGREDIENT | WEIGHT PER mL | |
|---|---|---|
| | 10.0 mg | PLACEBO |
| THERAPEUTIC COMPOUND (1) | 10.0 mg | -- |
| Dibasic Sodium Phosphate, Heptahydrate, USP | 11.97 mg | 10.74 mg |
| Monobasic Sodium Phosphate, Monohydrate, USP | 0.74 mg | 1.25 mg |
| Sodium Chloride, USP | 4.50 mg | 5.48 mg |
| 1 N Sodium Hydroxide Solution or 0.05 M Monobasic Sodium Phosphate Solution (2) | q.s. | q.s. |
| Water for Injection, USP | 1.0 mL | 1.0 mL |
| q.s. ad | (1.01 gm) | (1.01 gm) |

| | | |
|---|---|---|
| (1) The nominal concentration of THERAPEUTIC COMPOUND in this formulation is 10 mg/mL. A manufacturing adjustment is made for the drug substance purity. | | |
| (2) Added to adjust pH to 7.0-7.5 | | |

### MANUFACTURING DIRECTIONS: THERAPEUTIC SOLUTION

1. Charge approximately 90% of the required amount of Water for Injection, USP to a vessel equipped with a suitable agitation device, and connected to a heater/cooler circulation bath.
2. Adjust the temperature of the circulation bath to 30 °C.
3. Charge with continuous stirring, the required amount of Dibasic Sodium Phosphate, Heptahydrate, USP and continue stirring until dissolved.
4. Charge very slowly with continuous stirring the required amount of THERAPEUTIC COMPOUND.
5. Continue to stir for approximately 30 minutes until dissolved, then decrease the temperature of the circulation bath to 25 °C.
6. Charge with continuous stirring the required amount of Monobasic Sodium Phosphate, Monohydrate, USP and continue stirring until dissolved.
7. Charge with continuous stirring the required amount of Sodium Chloride, USP and continue stirring until dissolved.
8. Measure the pH and adjust to 7.0 to 7.5 with 1 N Sodium Hydroxide Solution or 0.05 M Monobasic Sodium Phosphate Solution, if necessary.
9. Bring the batch to final weight (calculated from specific gravity of 1.01) with Water for Injection, USP.
10. Aseptically filter the bulk solution into a suitable, sterilized filling vessel. Aseptically fill and seal the ampules.
11. Leak test ampules and visually inspect for particulate matter and other defects.

### MANUFACTURING DIRECTIONS: PLACEBO

The procedure listed above is carried out with the omission of steps 2, 4 and 5, and without the need for temperature control.

## Claims

1. The use of 4-(4-chlorophenylsulphonylcarbamoyl)benzoyl-L-valyl-L-proline 1(RS)-(1-trifluoroacetyl-2-methylpropyl)amide, or a pharmaceutically acceptable salt thereof, in the manufacture of a novel medicament for use in the treatment of bronchitis.

2. The use as claimed in claim I wherein the pharmaceutically acceptable salt of the acid is selected from alkali metal and alkaline earth metal salts, ammonium salts, and salts with organic bases affording a pharmaceutically acceptable cation.

3. The use as claimed in claim 1 or 2 wherein the pharmaceutically acceptable salt of the acid is a sodium or potassium salt.

4. The use as claimed in claim 1, 2 or 3 where in addition another pharmacological agent indicated for the treatment of bronchitis is used in the manufacture of the novel medicament.

5. The use as claimed in claim 4 wherein the other pharmacological agent is selected from antibiotics, bronchodilators, corticosteroids, oxygen, mucolytics and mucorheologic agents.

6. The use as claimed in any preceding claim wherein the novel medicament is in the form of an aerosol.

## Patentansprüche

1. Verwendung von 4-(4-Chlorphenylsulfonylcarbamoyl)benzoyl-L-valyl-L-prolin-1(RS)-(1-trifluoracetyl-2-methylpropyl)amid oder eines pharmazeutisch verträglichen Salzes davon zur Herstellung eines neuen Arzneimittels zur Verwendung bei der Behandlung von Bronchitits.

2. Verwendung nach Anspruch 1, wobei das pharmazeutisch verträgliche Salz der Säure aus Alkalimetall- und Erdalkalimetallsalzen, Ammoniumsalzen und Salzen mit organischen Basen, die ein pharmazeutisch verträgliches Kation besitzen, ausgewählt wird.

3. Verwendung nach Anspruch 1 oder 2, wobei das pharmazeutisch verträgliche Salz der Säure ein Natrium- oder Kaliumsalz ist.

4. Verwendung nach Anspruch 1, 2 oder 3, wobei zusätzlich ein anderer pharmakologischer Wirkstoff, der zur Behandlung von Bronchitis verwendet wird, zur Herstellung des neuen Arzneimittels verwendet wird.

5. Verwendung nach Anspruch 4, wobei der zusätzliche pharmakologische Wirkstoff aus Antibiotika, Bronchodilatatoren, Corticosteroiden, Sauerstoff zuführenden Substanzen, schleimlösenden Mitteln und mucorheologischen Mitteln ausgewählt wird.

6. Verwendung nach einem der vorstehenden Ansprüche, wobei das neue Arzneimittel in Form eines Aerosols vorliegt.

## Revendications

1. Utilisation du 1(RS)-(1-trifluoroacétyl-2-méthylpropyl)amide de 4-(4-chlorophénylsulfonylcarbamoyl)benzoyl-L-valyl-L-proline, ou d'un sel pharmaceutiquement acceptable de celui-ci, dans la fabrication d'un nouveau médicament destiné à être utilisé dans le traitement de la bronchite.

2. Utilisation selon la revendication 1, où le sel pharmaceutiquement acceptable de l'acide est choisi parmi les sels de métaux alcalins et de métaux alcalino-terreux, les sels d'ammonium et les sels avec des bases organiques donnant un cation pharmaceutiquement acceptable

3. Utilisation selon la revendication 1 ou 2, où le sel pharmaceutiquement acceptable de l'acide est un sel de sodium ou de potassium.

4. Utilisation selon la revendication 1, 2 ou 3, où, en outre, un autre agent pharmacologique indiqué pour le traitement de la bronchite est utilisé dans la imbrication du nouveau médicament.

5. Utilisation selon la revendication 4, où l'autre agent pharmacologique est choisi parmi les antibiotiques, les bronchodilatateurs, les corticostéroïdes, l'oxygene, les mucolytiques et les agents mucorhéologiques.

6. Utilisation selon l'une quelconque des revendications précédentes, où le nouveau médicament est sous forme d'un aérosol.
